(19) Europäisches Patentamt
European Patent Office
Office européen des brevets

(11) Veröffentlichungsnummer: **0 007 476**
**B1**

# (12) EUROPÄISCHE PATENTSCHRIFT

(45) Veröffentlichungstag der Patentschrift: **24.03.82**

(51) Int. Cl.³: **G 01 N 21/76,** C 12 Q 1/66

(21) Anmeldenummer: **79102245.2**

(22) Anmeldetag: **03.07.79**

(54) **Verfahren und Reagens zur Bestimmung eines oxidierten Pyridin-coenzyms.**

(30) Priorität: **01.08.78 DE 2833723**

(43) Veröffentlichungstag der Anmeldung: **06.02.80 Patentblatt 80/3**

(45) Bekanntmachung des Hinweises auf die Patenterteilung: **24.03.82 Patentblatt 82/12**

(84) Benannte Vertragsstaaten: **BE CH DE FR GB IT LU NL SE**

(56) Entgegenhaltungen:

**DE-A1-2 602 961**
**DE-A1-2 722 381**

**ANGEWANDTE CHEMIE, Band 89, 1977, Weinheim G. D. MENDENHALL «Analytische Anwendung der Chemilumineszenz» Seiten 220 bis 228**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Wulff, Karl, Dr., Pütrichstrasse 18, D-8120 Weilheim (DE)**
Erfinder: **Stähler, Fritz, Dr., Heimgartenstrasse 4, D-8132 Tutzing (DE)**
Erfinder: **Michal, Gerhard, Dr., Kreuzeckstrasse 19, D-8132 Tutzing (DE)**

(74) Vertreter: **Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

**EP 0 007 476 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren und Reagens zur Bestimmung eines oxidierten Pyridin-coenzyms

Die Erfindung betrifft ein Verfahren zur direkten Bestimmung eines oxidierten Pyridin-coenzyms mit Hilfe eines bakteriellen Biolumineszenzsystems und ein Reagens zur Durchführung dieses Verfahrens.

Unter den oxidierten Pyridin-coenzymen versteht man das Nicotinamid-adenin-dinucleotid (NAD) und das Nicotinamid-adenosin-dinucleotid-phosphat (NADP). Das bakterielle Biolumineszenzsystem besteht aus zwei Enzymen, einer Luciferase und einer Flavinmononucleotid-reduktase (FMN-Reduktase). Dieses System kommt in Fotobakterien, wie z.B. Photobacterium fischeri, Photobacterium phosphoreum oder Beneckea harveyi vor, und katalysiert die folgenden Reaktionen:

$$1)\ FMN + NAD(P)H \xrightleftharpoons{\text{FMN-Reduktase}} FMNH_2 + NAD(P)$$

$$2)\ FMNH_2 + O_2 + \text{Aldehyd} \xrightarrow{\text{Luciferase}} FMN + \text{Säure} + \text{Licht}\ \ (495\ nm)$$

NAD(P) bezeichnet hier ein oxidiertes Pyridin-coenzym,
NAD(P)H ein reduziertes Pyridin-coenzym.

Die Intensität des bei dieser Reaktion emittierten Lichts ist definiert als eine Geschwindigkeit

$$I = \frac{d(h \times v)}{dt}$$

und je nachdem, ob man die Reaktion erster oder pseudoerster Ordnung in bezug auf $FMNH_2$ oder den Aldehyd ablaufen lässt, ist die Lichtintensität der Konzentration des $FMNH_2$ oder der des Aldehyds proportional. In obiger Gleichung bedeuten: I = Lichtintensität, h = Planck'sches Wirkungsquantum, v = Frequenz.

Im bakteriellen Lumineszenzsystem existieren drei verschiedene FMN-Reduktasen, wovon eine ausschliesslich NADH-abhängig, eine zweite ausschliesslich NADPH-abhängig und die dritte sowohl NADH- als auch NADPH-abhängig ist.

Es ist bereits bekannt, das bakterielle Biolumineszenzsystem, meist in Form einer rohen Präparation, welche die Enzyme FMN-Reduktase und Luciferase gemischt enthält, zur quantitativen Bestimmung der reduzierten Pyridin-coenzyme, also NADH oder NADPH, zu verwenden. Ist dieses System mit Aldehyd und FMN gesättigt, so läuft die Gesamtreaktion bezüglich des Pyridin-coenzyms nahezu nach pseudoerster Ordnung ab, d.h. die Lichtintensität ist der Konzentration an reduziertem Pyridin-coenzym proportional. Ein Vorzug dieser Methode ist die ausserordentlich hohe Empfindlichkeit, die es erlaubt, die reduzierten Pyridin-coenzyme noch in Konzentrationen von $10^{-12}$ bis $10^{-13}$ Mol/l genau zu bestimmen. Nach diesem bekannten Verfahren lassen sich zwar auch die oxidierten Pyridin-coenzyme prinzipiell bestimmen, jedoch nur dann, wenn diese vorher vollständig in die reduzierten Pyridin-coenzyme umgewandelt worden sind. Dies stellt einen erheblichen Nachteil dar, wie im folgenden erläutert wird:

Die Bestimmung der oxidierten Pyridin-coenzyme besitzt bereits an sich Bedeutung im Rahmen von biochemischen Untersuchungen. Besonders wichtig ist jedoch die Bestimmung von intermediär im Rahmen vorgeschalteter, von Dehydrogenasen katalysierten Reaktionen gebildeter oxidierter Pyridin-coenzyme. Typische Beispiele hierfür sind die durch Lactatdehydrogenase, Malatdehydrogenase und Glutamat-dehydrogenase katalysierten, durch die folgenden Reaktionsgleichungen dargestellten Reaktionen:

$$3)\ \text{Pyruvat} + NADH \xrightleftharpoons{LDH} \text{Lactat} + NAD$$

$$4)\ \alpha\text{-Ketoglutarat} + NAD(P)H + NH_3 \xrightleftharpoons{\text{Glu-DH}} \text{1-Glu} + NAD(P)$$

$$5)\ \text{Oxalacetat} + NADH \xrightleftharpoons{MDH} \text{Malat} + NAD$$

Diese Reaktionen werden zur Bestimmung von Pyruvat, α-Ketoglutarat und Oxalacetat insbesondere in der klinischen Analyse in grossem Umfang angewendet. Bisher sind zwei Wege bekannt, diese Reaktionen mit Hilfe des bakteriellen Biolumineszenzsystems über die Bestimmung des gebildeten oxidierten Pyridin-coenzyms zu messen.

1. Man lässt die vorgeschaltete Dehydrogenasereaktion vollständig ablaufen, zerstört das überschüssige reduzierte Pyridin-coenzym chemisch vollständig, wobei das stabilere, durch

die Dehydrogenasereaktion gebildete oxidierte Pyridin-coenzym erhalten bleibt, führt danach letzteres in die reduzierte Form über und misst dann mit dem bakteriellen Biolumineszenzsystem (vgl. z.B. Analyt. Biochem. 78, 229-234). Dieses Verfahren ist jedoch sehr aufwendig und erlaubt es nicht, eine Dehydrogenasereaktion, die unter Bildung der oxidierten Pyridincoenzyme abläuft, kontinuierlich zu verfolgen.

2. Eine NAD(P)H-verbrauchende und NAD(P)-liefernde Reaktion kann prinzipiell auch über eine kontinuierliche oder diskontinuierliche Messund des NAD(P)H-Verbrauchs verfolgt werden. Aufgrund der enzymkinetischen Parameter der meisten Dehydrogenase-Reaktionen müsste dann hier jedoch eine derart hohe Ausgangskonzentration an NADH bzw. NADP wählen, dass messtechnisch das dieser Konzentration entsprechende durch das Biolumineszenzsystem generierte Lichtsignal in den üblicherweise für derartige Messungen verwendeten hochempfindlichen Messgeräten nicht mehr verarbeitet werden kann. Ausserdem ist aber die zu erwartende Signalabnahme im Vergleich zu der Höhe des Ausgangssignals so gering, dass sie in Messgeräten geringerer Empfindlichkeit nicht mehr gemessen werden kann. Wählt man eine entsprechend niedrige Coenzym-Konzentration, die messtechnisch noch zugänglich ist, so läuft die zu messende Dehydrogenase-Reaktion nicht optimal ab, man erhält keine verlässlichen Messwerte und hat ausserdem noch das messtechnische Problem, dass man, ausgehend von einer sehr hohen Anfangs-Lichtemission, einen verhältnismässig kleinen Signalabfall misst.

Der Erfindung liegt daher die Aufgabe zugrunde, diese Nachteile zu überwinden und einen direkten Weg zu finden, bei dem in einer gekoppelten enzymatischen Reaktion ein der Konzentration des oxidierten Pyridin-coenzyms entsprechendes, z.B. proportionales, Lichtsignal über einem dunklen Untergrund generiert wird.

Gelöst wird diese Aufgabe erfindungsgemäss durch ein Verfahren zur quantitativen Bestimmung eines oxidierten Pyridin-coenzyms unter Oxidation mit Sauerstoff in Gegenwart des Luciferasesystems und Messung des emittierten Lichts, welches dadurch gekennzeichnet ist, dass man das oxidierte Pyridin-coenzym in Gegenwart seiner spezifischen Alkoholdehydrogenase mit einem aliphatischen Alkohol mit 8 bis 16 C-Atomen umsetzt und den gebildeten Aldehyd mit reduziertem Flavin-mononucleotid ($FMNH_2$) und $O_2$ in Gegenwart von Luciferase und der von dem zu bestimmenden Coenzym jeweils unabhängigen FMN-Reduktase sowie dem reduzierten Substrat-coenzym der letzteren unter Lichtemission oxidiert.

Das Prinzip des Verfahrens besteht darin, dass der in der Luciferase katalysierten Reaktion umgesetzte höherkettige Aldehyd mit Hilfe einer Alkoholdehydrogenase aus dem entsprechenden Alkohol unter Reduktion des oxidierten Pyridin-coenzyms gebildet wird. Auf diese Weise ist es möglich, eine der Konzentration des oxidierten Pyridin-coenzyms entsprechende Konzentration an Aldehyd zu bilden, die ihrerseits ein ihr proportionales Lichtsignal hervorruft.

Aus der DE-A-2 722 391 ist es bereits bekannt, dass sich die Alkohol-Dehydrogenase-Reaktion mit Hilfe der FMN-Reduktase/Luciferase aus Photobakterien verfolgen lässt. Die Alkohol-Dehydrogenase-Reaktion wird dabei verwendet um Äthanol-Konzentrationen oder Alkohol-Dehydrogenase-Aktivitäten zu bestimmen. Die «Messgrösse» bei der bekannten Reaktionsfolge ist die NADH-Konzentration.

Erfindungsgemäss wird die Alkohol-Dehydrogenase verwendet, um mit Hilfe eines höherkettigen Alkohols eine der NAD-Konzentration proportionale Menge an korrespondierenden höherkettigen Aldehyd zu erzeugen. Die «Messgrösse» in der nachgeschalteten Biolumineszenz-Reaktion ist daher der Aldehyd.

Wie oben bereits erwähnt, lässt sich das erfindungsgemässe Verfahren dazu anwenden, bereits vorliegendes oxidiertes Pyridin-coenzym, also NAD oder NADP, zu bestimmen oder aber diese Bestimmung lediglich im Rahmen einer vorgeschalteten Dehydrogenase-Reaktion durchzuführen, in deren Verlauf das oxidierte Pyridincoenzym erst gebildet wird, und auf diese Weise ein Substrat oder Enzym der vorgeschalteten Reaktion zu messen. Bei der ersteren dieser Ausführungsformen ist es möglich, mit relativ rohen Enzympräparaten des bakteriellen Biolumineszenzsystems zu arbeiten, beispielsweise rohen oder nur wenig gereinigten Extrakten geeigneter Fotobakterien. In diesen rohen Präparationen ist die Luciferase und die FMN-Reduktase in einer für das Verfahren ausreichenden Konzentration und Aktivität vorhanden. Wird hingegen die zweite Ausführungsform des Verfahrens der Erfindung, also die Bestimmung eines oxidierten Pyridin-coenzyms, welches in situ im Rahmen einer vorgeschalteten Dehydrogenasereaktion gebildet wird, durchgeführt, so muss eine gereinigte FMN-Reduktase verwendet werden, und zwar jeweils eine solche, die von dem zu bestimmenden oxidierten Pyridin-coenzym unabhängig ist und keine FMN-Reduktase-Aktivität mehr enthält, die von diesem zu bestimmenden, oxidierten Pyridin-coenzym abhängig ist.

Ausserdem setzt man je nachdem, welches der beiden oxidierten Pyridin-coenzyme bestimmt wird, die jeweils von diesem Coenzym abhängige spezifische Alkoholdehydrogenase ein. Wird also NAD bestimmt, so wird die ADH (EC 1.1.1.1) verwendet, die beispielsweise aus Pferdeleber oder Hefe gewonnen wird. Bestimmt man NADP, so wird die NADP-abhängige Alkoholdehydrogenase (EC 1.1.1.2) verwendet, die sich beispielsweise aus Leuconostoc mesenteroides gewinnen lässt. Diese Enzyme sind bekannt und eine nähere Beschreibung erübrigt sich daher hier. Die von diesen zwei Typen von Alkoholdehydrogenasen katalysierten Reaktionen sind in den nachfolgenden Gleichungen 6) und 7) dargestellt.

$$6)\ NAD + Alkohol \xrightleftharpoons{ADH\ (EC\ 1.1.1.1)} Aldehyd + NADH$$

$$7)\ NADP + Alkohol \xrightleftharpoons{ADH\ (EC\ 1.1.1.2)} Aldehyd + NADPH$$

Aus Biochemistry 12, 4911-4918 (1973) war es bereits bekannt, dass ADH aus Pferdeleber n-Dekanol zu n-Dekanal umsetzt und letzterer mit Luciferase reagiert. Hieraus liess sich jedoch nicht entnehmen, dass es möglich wäre, eine von der NAD-Konzentration direkt abhängige Lichtemission in solcher Weise zu erzielen, dass eine quantitative Messung möglich ist.

Je nachdem, ob es sich bei dem zu bestimmenden oxidierten Pyridin-coenzym um NAD oder NADP handelt, ergeben sich damit folgende Reaktionsabläufe, dargestellt bei Verwendung von Dekanol:

NAD:

$$8)\ Dekanol + NAD \xrightleftharpoons{ADH\ (EC\ 1.1.1.1)} Dekanal + NADH$$

$$9)\ FMN + NADPH \xrightleftharpoons[FMN\text{-}Reduktase]{NADPH\text{-}abh\ddot{a}ngige} FMNH_2 + NADP$$

$$10)\ Dekanal + FMNH_2 + O_2 \longrightarrow FMN + Dekans\ddot{a}ure + Licht$$

NADP:

$$11)\ Dekanol + NADP \xrightleftharpoons{ADH\ (EC\ 1.1.1.2)} Dekanal + NADPH$$

$$12)\ FMN + NADH \xrightleftharpoons[FMN\text{-}Reduktase]{NADH\text{-}abh\ddot{a}ngige} FMNH_2 + NAD$$

$$13)\ Dekanal + FMNH_2 + O_2 \longrightarrow FMN + Dekans\ddot{a}ure + Licht$$

Bei Verwendung des rohen FMN-Reduktasepräparats, welches in der Regel alle drei FMN-Reduktasen enthält, wird die Spezifität in diesem Falle durch Wahl des entsprechenden reduzierten Pyridin-coenzyms erzielt, bei NAD also NADPH und bei NADP dementsprechend NADH, so dass Störungen durch die Reaktion 9) bzw. 12) vermieden werden.

Bei der zweiten Ausführungsform des erfindungsgemässen Verfahrens, also mit vorgeschalteter Dehydrogenasereaktion, ist es jedoch erforderlich, in der vorgeschalteten Reaktion ein reduziertes Pyridin-coenzym einzusetzen, was durch folgende Gleichung veranschaulicht wird:

$$14)\ Substrat + NAD(P)H \xrightleftharpoons{Dehydrogenase} Produkt + NAD(P)$$

In den Reaktionen 8) bzw. 11) wird jedoch das gleiche reduzierte Pyridin-coenzym gebildet.

Man erkennt, dass jetzt die Gefahr besteht, dass durch in Reaktion 9) bzw. 12) gebildetes oxidiertes Pyridin-coenzym stört und die Reaktion 8) bzw. 11) im Gleichgewicht gestört werden kann, wenn hier alle drei FMN-Reduktasen wirksam werden können. Daher wird in diesem Falle ein FMN-Reduktasepräparat eingesetzt, welches nur noch Aktivität für eines der beiden Pyridin-coenzyme aufweist.

Das erfindungsgemässe Verfahren wird in gepufferter Lösung durchgeführt, wobei im allgemeinen pH-Werte zwischen 6 und 9 geeignet sind. Die Wahl des pH-Wertes richtet sich nach den jeweils eingesetzten Enzymen. Bei den Alkoholen lassen sich die niedrigeren Glieder noch als wässrige Lösungen einsetzen, die höheren hingegen weisen eine so geringe Löslichkeit auf, dass sie in Form von Suspensionen, die beispielsweise durch Ultraschallbehandlung erhalten werden, eingesetzt werden. Zweckmässig werden dann auch oberflächenaktive Stoffe zur Stabilisierung dieser Suspensionen zugesetzt. Bevorzugt werden als oberflächenaktive Mittel die nicht-ionischen, insbesondere die Alkarylpolyoxyäthylen-ester und -äther. Dies gilt insbesondere für Tetradekanol und Hexadekanol,

aber auch bei Dodekanol kommt die Anwendung als Suspension in Betracht. Die im Test einzusetzenden Alkoholmengen liegen, bezogen auf ml Testlösung, zwischen 0,01 und 25 mM. Bei rohen Luciferase/FMN-Reduktase-Präparaten betragen die entsprechenden Mengen 0,5 bis 50 µg. Werden die gereinigten Enzyme eingesetzt, so wendet man die dieser Gewichtsmenge entsprechenden Aktivitätsmengen an. Bei der ADH sind die eingesetzten Aktivitäten stark von der Quelle des Enzyms abhängig und liegen im allgemeinen zwischen 0,05 und 300 mU. Das jeweils zugesetzte reduzierte Pyridincoenzym wird zweckmässig im Mengen zwischen 1 und 250 × $10^{-6}$ M zugegeben.

Ausserdem kann es zweckmässig sein, eine organische Mercapto-Verbindung zuzusetzen. Hier sind Mengen von 0,002 bis 0,5 mM zweckmässig. Die anwendbare Pufferkonzentration schliesslich liegt zwischen etwa 2 und 130 mM.

Ein weiterer Gegenstand der Erfindung ist ein Reagens zur Bestimmung eines oxidierten Pyridin-coenzyms, welches gekennzeichnet ist durch einen Gehalt an Luciferase, FMN-Reduktase, einem aliphatischen Alkohol mit 8 bis 16 C-Atomen, einem reduzierten Coenzym, einer für das zu bestimmende Coenzym spezifischen Alkoholdehydrogenase, Flavin-mononucleotid (FMN), Puffer und gegebenenfalls einer organischen Mercaptoverbindung oder/und einem oberflächenaktiven Mittel.

Gemäss einer bevorzugten Ausführungsform enthält dieses Reagens

| | |
|---|---|
| 0,5 bis 50 µg | Luciferase/FMN-Reduktase-präparat |
| 0,5 bis 300 mU | Alkoholdehydrogenase |
| 0,01 bis 25 mM | aliphatischen Alkohol mit 8 bis 16 C-Atomen |
| 2 bis 130 mM | Puffer, pH 6 bis 9 |
| 1 bis 250 × $10^{-6}$ M | reduziertes Pyridin-coenzym |
| und gegebenenfalls | |
| 0,002 bis 0,5 mM | organische Mercaptoverbindung/ml Testvolumen. |

Besonders bevorzugt wird eine Reagentienzusammensetzung, welche

| | |
|---|---|
| 1,8 bis 40 µg | Luciferase/FMN-Reduktase-präparat |
| 1,3 bis 2,7 mU | Alkoholdehydrogenase |
| 0,1 bis 10 mM | Alkohol |
| 5 bis 50 mM | Puffer |
| 5 bis 150 × $10^{-6}$ M | reduziertes Pyridin-coenzym |
| und gegebenenfalls | |
| 0,005 bis 0,5 mM | organische Mercaptoverbindung/ml Testvolumen |

enthält.

Als Mercaptoverbindung eignet sich besonders Mercaptoäthanol, aber auch andere organische Mercaptoverbindung, wie z.B. Dithiothreit, Dithioerythrit, reduziertes Glutathion, NAC (N-Acetylcystein) sind geeignet.

Zur weiteren Erläuterung dient die beigefügte Zeichnung in Verbindung mit den Beispielen. In der Zeichnung stellen dar:

Fig. 1 eine graphische Darstellung der Relation von maximaler Lichtintensität zu NAD-Konzentration bei dem Verfahren von Beispiel 1.

Fig. 2 eine graphische Darstellung der Relation von maximaler Lichtintensität zu NAD-Konzentration gemäss Beispiel 2.

Fig. 3 eine graphische Darstellung entsprechend Fig. 1 und 2 unter Verwendung eines längerkettigen Alkohols gemäss Beispiel 3.

Fig. 4 eine graphische Darstellung entsprechend den Fig. 1 bis 3 für die Messung der NADP-Konzentration gemäss Beispiel 4.

Die Erfindung schafft erstmalig ein äusserst empfindliches und genaues Verfahren zur Messung eines oxidierten Pyridin-coenzyms mit Hilfe des bakteriellen Lumineszenzsystems, welches sich rasch und einfach durchführen lässt, auch mit vorgeschalteten Dehydrogenasereaktionen zusammen angewendet werden kann und sich auch mit relativ einfachen und nicht besonders hochempfindlichen Messgeräten durchführen lässt.

Die folgenden Beispiele erläutern dies weiter. In diesen Beispielen wurden alle Lichtmessungen unter Verwendung des ATP-Photometers, Modell 3000 der Fa. SAI Technology Co., San Diego, Calif., USA, durchgeführt. Als Puffer wurde jeweils Kaliumphosphatpuffer eingesetzt. Weitere besonders gut geeignete Puffer sind Trisacetat, Triäthanolaminhydrochlorid, Imidazolacetat. Auch Trissulfat kann verwendet werden.

Beispiel 1

Bestimmung von NAD-Konzentrationen mit Pferdeleber-ADH (EC 1.1.1.1)

Reaktionsbedingungen: Volumen 2,0 ml

Enkonzentration im Test:

80 mM Kaliumphosphatpuffer, pH 7,0
0,1 mM Mercaptoäthanol
2,5 µM FMN
8 mM Dekanol
34 µg Luciferase/FMN-Reduktase
(Rohfraktion aus Photobacterium fischeri)
5,4 mU Alkoholdehydrogenase aus Pferdeleber
(spezifische Aktivität z.B. 2,7 U/mg)
$10^{-4}$ M NADPH
$10^{-4}$ bis $10^{-7}$ M NAD

Die Reagentien werden zusammengegeben bis auf die Pyridin-coenzyme. Dann wird mit NAD vorgestartet und exakt 15 s nach der NAD-Zugabe mit NADPH gestartet. Die Lichtintensität steigt langsam an. Das Maximum der Intensität wird nach 3 bis 5 min erreicht. Die maximale Intensität ist die Messgrösse. Die Bestimmung kann auch so durchgeführt werden, dass mit einem Gemisch der beiden Pyridin-coenzyme, NAD und NADPH, gleichzeitig gestartet wird.

Die Fig. 1 zeigt die Relation von maximaler Lichtintensität zur NAD-Konzentration. Kontrollwerte ohne NAD wurden gemessen und deren Lichtintensität jeweils vom Messwert abgezogen.

Beispiel 2

Messung von NAD-Konzentrationen mit Hefe-ADH und Dekanol

Reaktionsbedingungen, wenn nicht anders vermerkt, wie bei Beispiel 1.

Anstelle von Pferde-ADH wurden 0,18 U/Test ADH aus Hefe verwendet mit einer spezifischen Aktivität von z.B. 300 U/mg.

Die Versuchsergebnisse sind in Fig. 2 dargestellt. Schwankungsbreiten für Hefe-ADH-Konzentration:
0,05 bis 18 U.

Beispiel 3

Bestimmung von NAD-Konzentrationen in Gegenwart von ADH aus Hefe oder Pferdeleber mit Hilfe höherkettiger Alkohole, wie z.B. Dodekanol und Tetradekanol

Bedingungen wie in den Beispielen 1 und 2. Anstelle einer Dekanol-Lösung wurden Ultraschallsuspensionen von Dodekanol oder Tetradekanol verwendet. Die Stammlösung waren 0,4 M in Wasser + 0,1% Triton X-100®. Von dieser Suspension wurden 20 µl in den Test eingesetzt. Die Endkonzentration im Test ist damit 4 mM an Dodekanol oder Tetradekanol. Fig. 3 zeigt die hier mit Tetradekanol und Pferdeleber-ADH erhaltenen Messwerte.

Beispiel 4

Bestimmung von NADP-Konzentrationen mit der ADH aus Leuconostoc mesenteroides (EC 1.1.1.2)

Versuchsdurchführung wie in den Beispielen 1 bis 3 beschrieben. Anstelle von NADPH wurde NADH eingesetzt.
Konzentration: $10^{-5}$ M
Grenzen: $10^{-6}$ bis $10^{-4}$ M.

NADP wurde bestimmt im Konzentrationsbereich von $10^{-7}$ bis $10^{-4}$ M.
ADH aus Leuconostoc:
Konzentration: 6 U/Test
Grenzen: 0,1 U bis 30 U/Test.

Fig. 4 zeigt die unter diesen Bedingungen bei Verwendung von Dekanol erhaltenen Versuchsergebnisse.

**Patentansprüche**

1. Verfahren zur Quantitativen Bestimmung eines oxidierten Pyridin-coenzyms unter Oxidation mit Sauerstoff in Gegenwart des Luciferasesystems und Messung des emittierten Lichts, dadurch gekennzeichnet, dass man das oxidierte Pyridin-coenzym in Gegenwart seiner spezifischen Alkoholdehydrogenase mit einem aliphatischen Alkohol mit 8 bis 16 C-Atomen umsetzt und den gebildeten Aldehyd mit reduziertem Flavin-mononucleotid ($FMNH_2$) und $O_2$ in Gegenwart von Luciferase und der von dem zu bestimmenden Coenzym jeweils unabhängigen FMN-Reduktase sowie dem reduzierten Substrat-coenzym der letzteren unter Lichtemission oxidiert.

2. Verfahren nach Anspruch 1, dadurch gekennzeichnet, dass man zur Konzentrationsbestimmung einer bereits vorliegenden Menge eines oxidierten Pyridin-coenzyms ein rohes Fotobakterienpräparat verwendet, welches Luciferase und ein Gemisch der FMN-Reduktasen enthält.

3. Verfahren nach Anspruch 1, dadurch gekennzeichnet, dass man zur Bestimmung eines oxidierten Pyridin-coenzyms, welches gleichzeitig in einer vorgeschalteten Dehydrogenasereaktion gebildet wird, gereinigte Luciferase und ein FMN-Reduktasepräparat verwendet, welches keine FMN-Reduktase enthält, die von dem zu bestimmenden oxidierten Pyridin-coenzym abhängig ist.

4. Verfahren nach einem der Ansprüche 1 bis 3, dadurch gekennzeichnet, dass man zur Bestimmung von NAD Alkoholdehydrogenase aus Pferdeleber oder Hefe verwendet.

5. Verfahren nach einem der Ansprüche 1 bis 3, dadurch gekennzeichnet, dass man zur Bestimmung von NADP Alkoholdehydrogenase aus Leuconostoc mesenteroides verwendet.

6. Reagens zur Bestimmung eines oxidierten Pyridin-coenzyms, gekennzeichnet durch einen Gehalt an Luciferase, FMN-Reduktase, einem aliphatischen Alkohol mit 8 bis 16 C-Atomen, einem reduzierten Coenzym, einer für das zu bestimmende Coenzym spezifischen Alkoholdehydrogenase, Flavin-mononucleotid (FMN), Puffer und gegebenenfalls einer organischen Mercaptoverbindung oder/und einem oberflächenaktiven Mittel.

7. Reagens nach Anspruch 6, dadurch gekennzeichnet, dass es

| | |
|---|---|
| 0,5 bis 50 µg | Luciferase/FMN-Reduktasepräparat |
| 0,5 bis 300 mU | Alkoholdehydrogenase |
| 0,01 bis 25 mM | aliphatischen Alkohol mit 8 bis 16 C-Atomen |
| 2 bis 130 mM | Puffer, pH 6 bis 9 |
| 1 bis 250 × $10^{-6}$ M | reduziertes Pyridin-coenzym |
| und gegebenenfalls | |
| 0,002 bis 0,5 mM | organische Mercaptoverbindung/ml Testvolumen |

enthält.

8. Reagens nach Anspruch 7, dadurch gekennzeichnet, dass es

| | |
|---|---|
| 1,8 bis 40 µg | Luciferase/FMN-Reduktasepräparat |
| 1,3 bis 2,7 mU | Alkoholdehydrogenase |
| 0,1 bis 10mM | Alkohol |
| 5 bis 50 mM | Puffer |
| 5 bis 150 × $10^{-6}$ M | reduziertes Pyridin-coenzym |
| und gegebenenfalls | |
| 0,005 bis 0,5 mM | organische Mercaptoverbindung/ml Testvolumen |

enthält.

9. Reagens nach einem der Ansprüche 6 bis 8, dadurch gekennzeichnet, dass es als Mercaptoverbindung Mercaptoäthanol enthält.

10. Reagens nach einem der Ansprüche 6

bis 9, dadurch gekennzeichnet, dass es als oberflächenaktives Mittel einen Aralkyl-polyoxyäthylen-ester oder -äther enthält.

**Claims**

1. Process for the quantative determination of an oxidized pyridin coenzyme by oxidation with oxygen in the presence of the luciferase system and by measurement of the light emitted, wherein the oxidized pyridin coenzyme is reacted in the presence of its specific alcohol-hydrogenase with an aliphatic alcohol having 8 to 16 C atoms and the aldehyde thus formed is oxidized with reduced flavin-mononucleotide ($FMNH_2$) and $O_2$ in the presence of lucerifase and of the FMN reductase which is respectively independent of the coenzyme to be determined as well as of the reduced substrate coenzyme of the latter with light emission.

2. Process according to claim 1, wherein, in order to determine the concentration of a quantity already present of an oxidized pyridin coenzyme, a raw photobacteria preparation is used, which contains lucerifase and a mixture of the FMN reductases.

3. Process according to claim 1, whereby in order to determine an oxidized pyridin coenzyme, which is simultaneously formed in a preceding dehydrogenase reaction, purified lucerifase and an FMN reductase preparation are used, which contains no FMN reductase which is dependent on the oxidized pyridin coenzyme to be determined.

4. Process according to one of claims 1 to 3 above, wherein alcohol hydrogenase from horse liver or yeast is used to determine NAD.

5. Process according to claims 1 to 3 above, wherein to determine NADP alcohol hydrogenase from leuconostoc mesenteroides is used.

6. Reagent for the determination of an oxidized pyridin coenzyme comprising a content of lucerifase, FMN reductase, an aliphatic alcohol having 8 to 16 C atoms, a reduced coenzyme, a specific alcohol dehydrogenase for the coenzyme to be determined, flavin-mononucleotide (FMN), buffer and optionally an organic mercapto compound and/or a surface active agent.

7. Reagent according to claim 6 above, wherein the said reagent contains:

| | |
|---|---|
| 0.5 to 50 µg | lucerifase/FMN reductase preparation |
| 0.5 to 300 mU | alcoholdehydrogenase |
| 0.01 to 25 mM | aliphatic alcohol having 8 to 16 C atoms |
| 2 to 130 mM | buffer, pH 6 to 9 |
| 1 to 250 × $10^{-6}$ M | reduced pyridin coenzyme |
| and optionally | |
| 0.002 to 0.5 mM | organic mercapto compound/ml of test volume. |

8. Reagent according to claim 7 above, wherein said reagent contains:

| | |
|---|---|
| 1.8 to 40 µg | lucerifase/FMN reductase preparation |
| 1.3 to 2.7 mU | alcohol dehydrogenase |
| 0.1 to 10 mM | alcohol |
| 5 to 50 mM | buffer |
| 5 to 150 × $10^{-6}$ M | reduced pyridin coenzyme |
| and optionally | |
| 0.005 to 0.5 mM | organic mercapto compound/ml of test volume. |

9. Reagent according to one of the claims 6 to 8 above, whereby said reagent contains mercaptoethanol as the mercapto compound.

10. Reagent according to one of the claims 6 to 9 above, whereby said reagent contains as the surface active agent an aralkyl-polyoxyethylene ester or an aralkyl-polyoxyethylene ether.

**Revendications**

1. Procédé pour la détermination quantitative d'un coenzyme de pyridine oxydé avec oxydation par l'oxygène en présence du système de luciférase et mesure de la lumière émise, caractérisé en ce que l'on fait réagir le coenzyme de pyridine oxydé en présence de son alcool déshydrogénase spécifique avec un alcool aliphatique avec 8 à 16 atomes de C et que l'on oxyde l'aldéhyde formé par du flavine-mononucléotide réduit ($FMNH_2$) et $O_2$ en présence de luciférase et de la FMN-réductase indépendante chaque fois du coenzyme à déterminer ainsi que du coenzyme-substrat réduit de ce dernier avec émission de lumière.

2. Procédé selon la revendication 1, caractérisé en ce que l'on utilise pour la détermination de la concentration d'une quantité déjà présente d'un coenzyme de pyridine oxydé, une préparation de photobactéries brute qui contient de la luciférase et un mélange des FMN-réductases.

3. Procédé selon la revendication 1, caractérisé en ce que l'on utilise pour la détermination d'un coenzyme de pyridine oxydé qui est formé simultanément dans une réaction de déshydrogénase pré-intercalée, de la luciférase purifiée et une préparation de FMN-réductase qui ne contient pas de FMN-réductase qui dépend du coenzyme de pyridine oxydé à doser.

4. Procédé selon l'une des revendications 1 à 3, caractérisé en ce que l'on utilise pour la détermination du NAD une alcool déshydrogénase à partir du foie de cheval ou de levure.

5. Procédé selon l'une quelconque des revendications 1 à 3, caractérisé en ce que l'on utilise pour la détermination du NADP une alcool déshydrogénase à partir de Leuconostoc mesenteroides.

6. Réactif pour la détermination d'un coenzyme de pyridine oxydé, caractérisé par une teneur en luciférase, en FMN-réductase, en alcool aliphatique avec 8 à 16 atomes de C, en un coenzyme réduit, en une alcool déshydrogénase spécifique pour le coenzyme à déterminer, en flavine-mononucléotide (FMN), en tampon et le

cas échéant en un composé organique mercapto et/ou en un agent tensio-actif.

7. Réactif selon la revendication 6, caractérisé en ce qu'il contient:

| | |
|---|---|
| 0,5 à 50 µg | de luciférase/préparation de FMN-réductase |
| 0,5 à 300 mU | d'alcool déshydrogénase |
| 0,01 à 25 mM | d'alcool aliphatique avec 8 à 16 atomes de C |
| 2 à 130 mM | de tampon, pH 6 à 9 |
| 1 à 250 × $10^{-6}$ M | de coenzyme de pyridine réduit |
| et le cas échéant | |
| 0,002 à 0,5 mM | de composé organique mercapto/ml du volume d'essai. |

8. Réactif selon la revendication 7, caractérisé en ce qu'il contient:

| | |
|---|---|
| 1,8 à 40 µg | de luciférase/préparation de FMN-réductase |
| 1,3 à 2,7 mU | d'alcool déshydrogénase |
| 0,1 à 10 mM | d'alcool |
| 5 à 50 mM | de tampon |
| 5 à 150 × $10^{-6}$ M | de coenzyme de pyridine réduit |
| et le cas échéant | |
| 0,005 à 0,5 mM | de composé organique mercapto/ml du volume d'essai. |

9. Réactif selon l'une des revendications 6 à 8, caractérisé en ce qu'il contient comme composé mercapto du mercaptoéthanol.

10. Réactif selon l'une des revendications 6 à 9, caractérisé en ce qu'il contient comme agent tensio-actif un ester ou éther d'aralcoylpolyoxyéthylène.

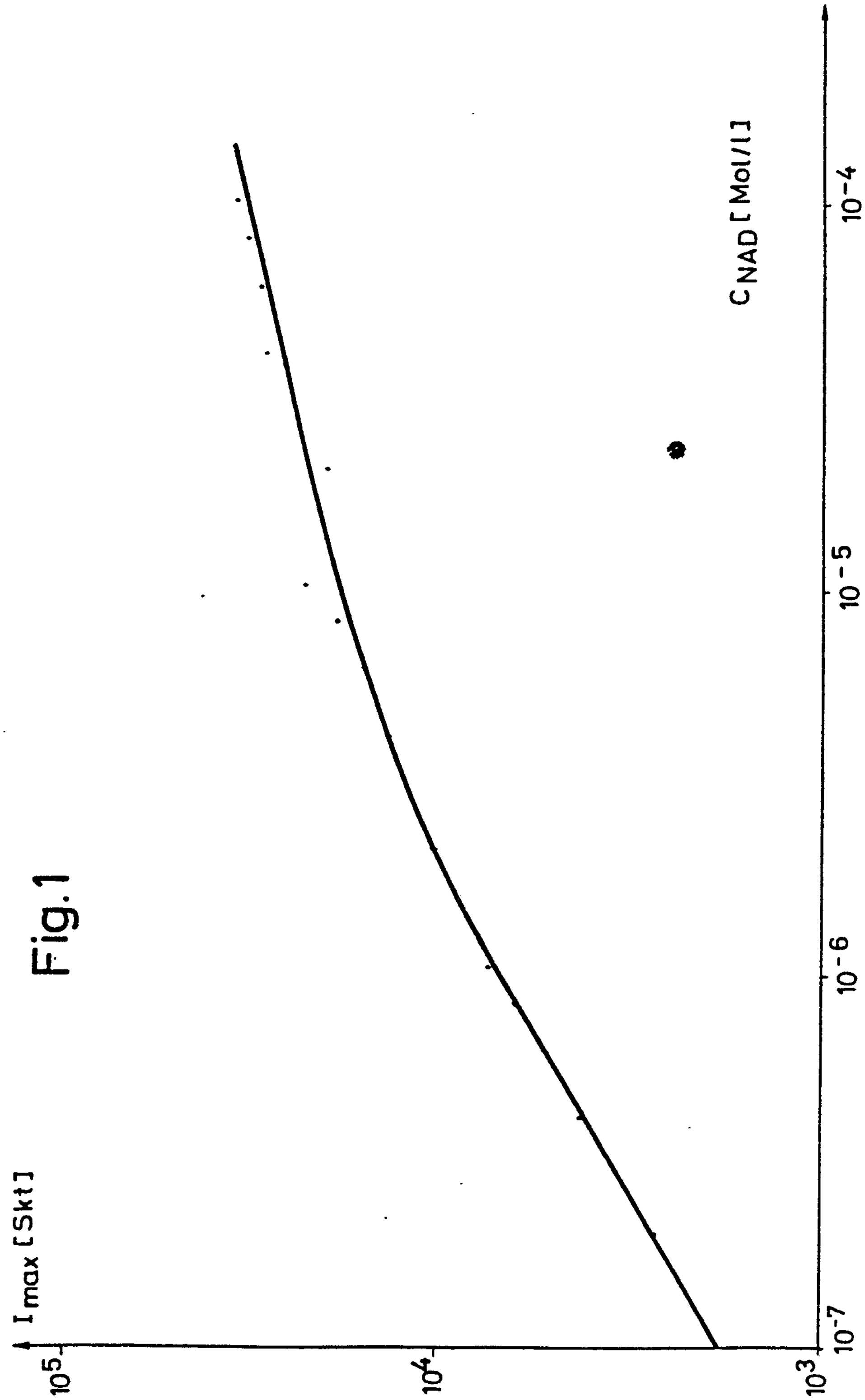


Fig.1

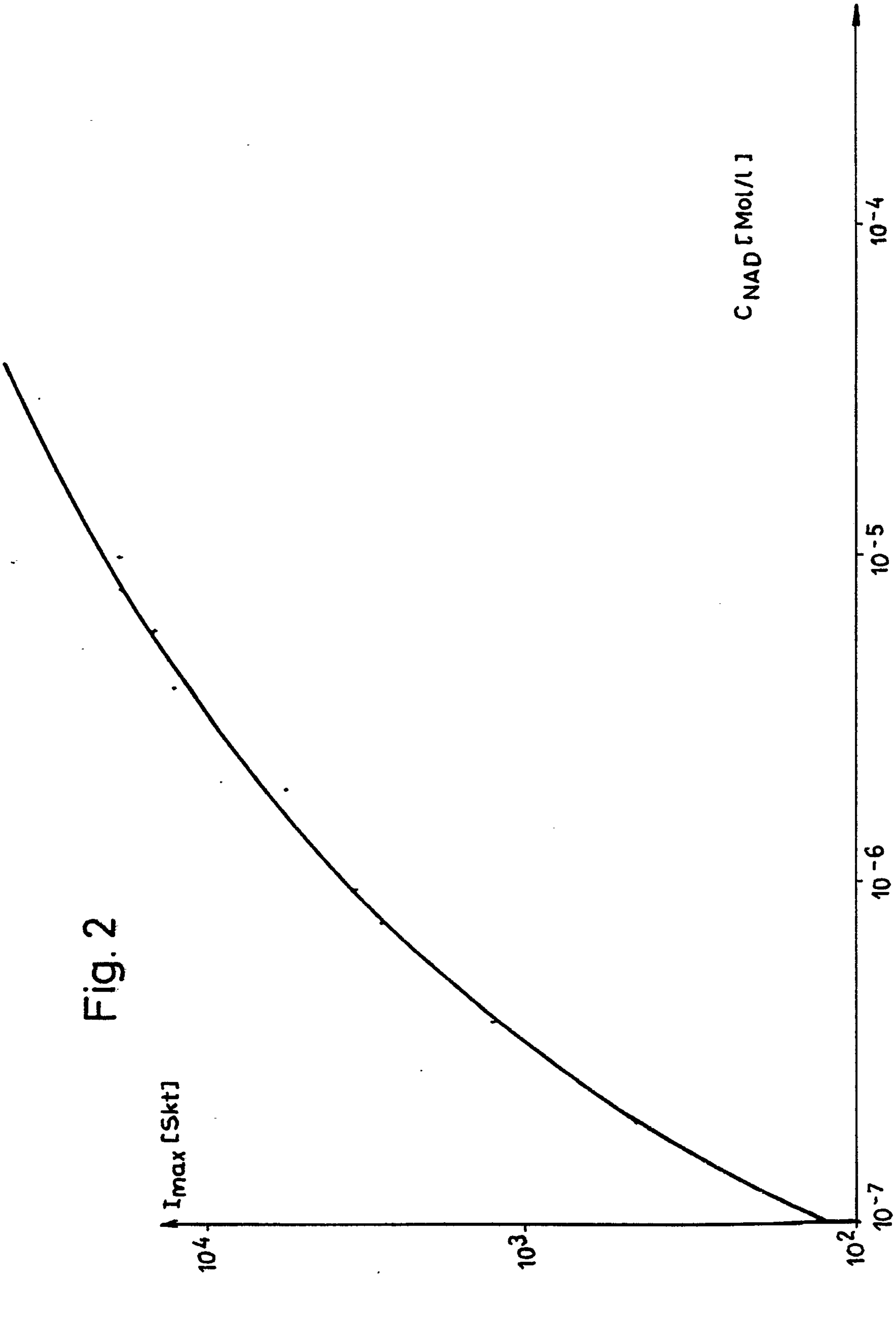


Fig. 2

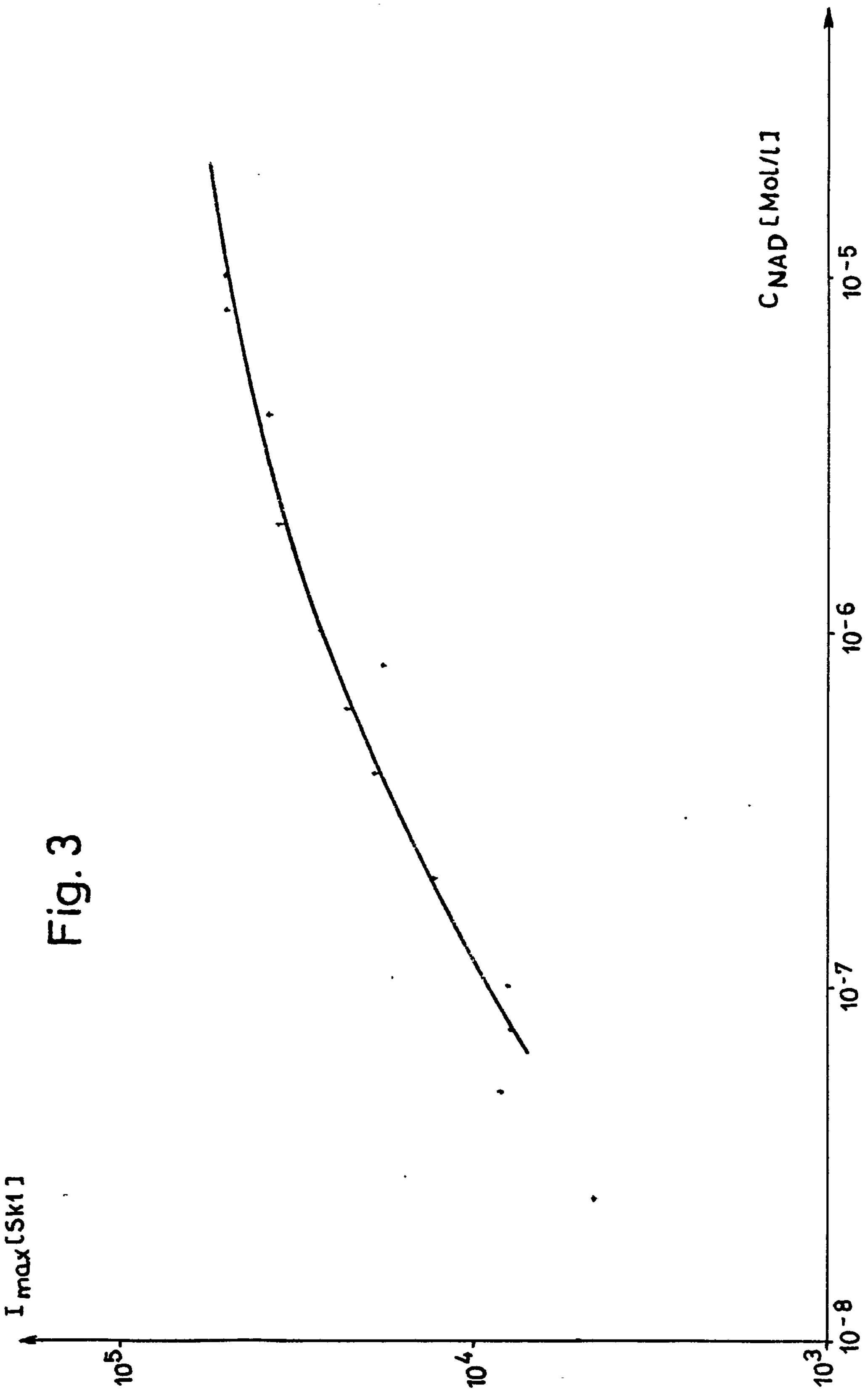


Fig. 3

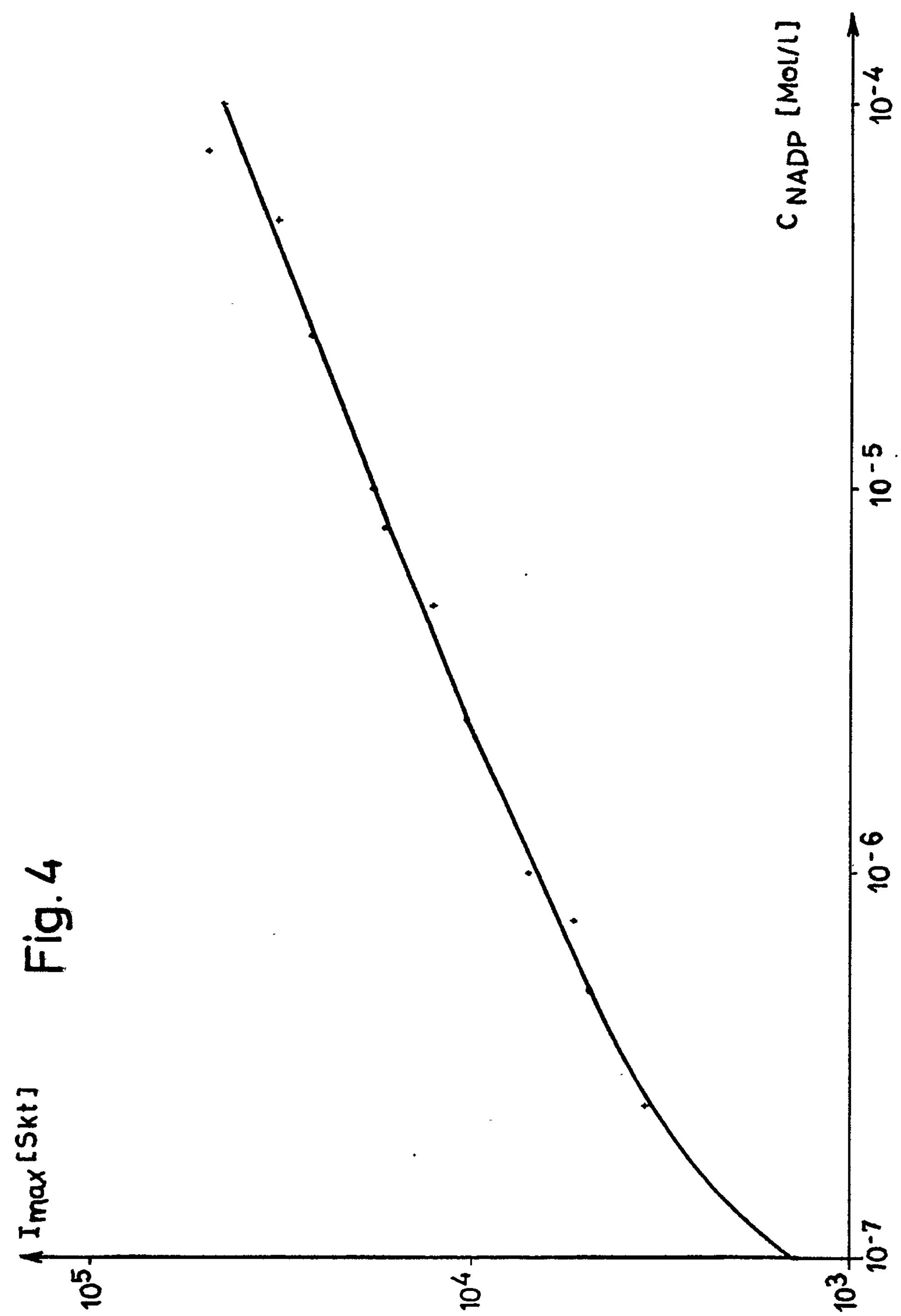


Fig. 4